Europäisches Patentamt

European Patent Office

Office européen des brevets

(19)

(11) Veröffentlichungsnummer: **0 331 861**

**A1**

(12) # EUROPÄISCHE PATENTANMELDUNG

(21) Anmeldenummer: 88810154.0

(22) Anmeldetag: 11.03.88

(51) Int. Cl.⁴: **G02F 1/13 , A61F 9/06**

(43) Veröffentlichungstag der Anmeldung:
13.09.89 Patentblatt 89/37

(84) Benannte Vertragsstaaten:
AT BE CH DE ES FR GB GR IT LI LU NL SE

(71) Anmelder: OPTREL AG
Ebnater Strasse 84
CH-9630 Wattwil(CH)

(72) Erfinder: Stanelli, Daniel
Kapplerstrasse 70
CH-9642 Ebnat Kappel(CH)
Erfinder: Fürthbauer, Rupert
Hauptgasse 30
CH-9620 Lichtensteig(CH)

(74) Vertreter: Rottmann, Maximilian R.
c/o Rottmann, Maspoli + Zimmermann AG
Glattalstrasse 37
CH-8052 Zürich(CH)

(54) Vorrichtung zur Steuerung der Transmission eines Lichtschutzfilters.

(57) Ein Lichtschutzfilter weist ein bezüglich seiner optischen Transmission regelbares Filterelement (4) auf. Zur Steuerung des Filterelementes ist einerseits eine manuelle Regelung (3) vorhanden, die unabhängig von der Intensität des einfallenden Lichtes ist, und eine automatische, von der Intensität des einfallenden Lichtes abhängige Steuerung (2). Die automatische Steuerung (2) wird erst oberhalb eines bestimmten Grenzwertes der Lichtintensität wirksam, in der Weise, dass bei starker Lichtintensität durch die manuelle Regelung (3) keine vollständige Hellsteuerung des Filterelementes (4) mehr eingestellt werden kann. Bei einer praktischen Realisation kann die Stromversorgungseinheit (1) sowohl eine galvanische Batterie als auch eine Solarzelle enthalten, die parallelgeschaltet und voneinander entkoppelt sind.

FIG.1

EP 0 331 861 A1

## Vorrichtung zur Steuerung der Transmission eines Lichtschutzfilters

Die Erfindung betrifft eine Vorrichtung zur Steuerung der Transmission eines Lichtschutzfilters, welches ein optoelektronisches Filterelement aufweist. Lichtschutzfilter dieser Art werden zum Schutz empfindlicher Empfänger gegen einfallendes Licht mit schädlicher Intensität, z.B. zum Schutz der Augen bei Schweissarbeiten, angewandt.

Aus der DE-OS 24 42 998 ist eine Sichtscheibe mit automatischer Regelung der Lichtdurchlässigkeit bekannt, welche sich für den Einsatz in den bei Schweissarbeiten zu verwendenden Schutzschild eignet und als optoelektronisches Filterelement eine Flüssigkristallzelle aufweist. Die hinter der Sichtscheibe ankommende Lichtmenge wird von einem Photosensor gemessen und als Regelgrösse einer Schaltung zugeführt, welche die Steuerspannung für die Flüssigkristallzelle derart einstellt, dass das durchgelassene Licht einem eingegebenen Sollwert entsprechend konstant gehalten wird. Für die Energieversorgung der Sichtscheibe und der elektronischen Regelschaltung ist ein photoelektrischer Wandler am Rande der Sichtscheibe vorgesehen.

In der Schweisstechnik haben sich jedoch Lichtschutzfilter mit automatischer Regelung der Transmission mangels genügender Anpassungsfähigkeit als unpraktisch erwiesen. Insbesondere stören die den unter Umständen starken Schwankungen in den Lichtverhältnissen der Umgebung unmittelbar folgenden Wechsel im Transmissionsgrad. Bevorzugt werden deshalb in diesem Anwendungsbereich Lichtschutzfilter mit manueller Steuerung der Transmission. Damit lässt sich die beim Schweissvorgang erforderliche Abdunkelung je nach Schweissverfahren, Umgebungslicht und persönlichem Blendempfinden individuell einstellen und der Schweissvorgang genauer beobachten.

Allerdings besteht in der Anwendung von manuellen Regelungen der Transmission allgemein die Gefahr einer unbeabsichtigten Fehleinstellung, die unter Umständen zu einer übermässig starken Lichteinstrahlung und damit zu einer störenden oder gar schädlichen Blendung der Augen führen kann. Solche Fehleinstellungen sind z.B. möglich durch äussere Einwirkung z.B. von Hindernissen auf das Stellglied während eines Schweissvorganges oder dadurch, dass der Schweisser während einer Beobachtungsphase unvermittelt auf eine stärkere Lichtquelle trifft.

Die Erfindung hat zur Aufgabe, eine Steuervorrichtung zu schaffen, welche unter Vermeidung der beschriebenen Nachteile sowohl eine weitreichende Anpassung des Lichtschutzfilters an die Anwenderbedürfnisse ermöglicht als auch eine optimale Sicherheit gegen unbeabsichtigten, übermässig starken Lichtdurchlass gewährleistet.

Die erfindungsgemässe Steuervorrichtung ist dadurch gekennzeichnet, dass eine von der Intensität des einfallenden Lichtes unabhängige, manuelle Steuerung und eine von der Intensität des einfallenden Lichtes abhängige, automatische Steuerung des optoelektronischen Filterelements vorgesehen sind, wobei die automatische Steuerung in einem Bereich oberhalb eines bestimmten Grenzwertes der Lichtintensität wirksam ist, und dass sich die Wirkungen der beiden Steuerungen in diesem Bereich derart überlagern, dass bei Lichtintensitäten über dem Grenzwert die Wirkung der manuellen Steuerung bei einer Betätigung im Sinne einer Erhöhung der Transmission durch die Wirkung der automatischen Steuerung kompensiert wird.

Im Bereich unterhalb des genannten Grenzwertes der Lichtintensität ist eine manuelle Steuerung anwendbar, die es ermöglicht, die Transmission des optoelektronischen Filterelementes zwischen einem Minimalwert und einem Maximalwert zu verändern, unabhängig von der in diesem Bereich herrschenden Lichtintensität. Bei Verwendung eines optoelektronischen Filterelementes mit von der angelegten Spannung gegenläufig abhängiger Transmission ist der Einstellbereich der manuellen Steuerung durch einen Spannungsbereich festgelegt, wobei dem Spannungswert Null der Maximalwert der Transmission entspricht. Durch Verlagerung dieses Spannungsbereiches auf ein höheres Potential lässt sich erreichen, dass der Einstellbereich bei einem endlichen, unteren Spannungswert, der also über dem Wert Null liegt, endet, so dass mit der manuellen Steuerung die Transmission nicht mehr auf ihren Maximalwert einstellbar ist.

Eine bevorzugte Lösung besteht in diesem Zusammenhang darin, dass die Stromversorgungseinheit der Vorrichtung eine die Steuerung des optoelektronischen Filterelementes bewirkende Spannung liefert, deren Wert von einer bestimmten Intensität des einfallenden Lichtes an mit der Lichtintensität zunimmt. Dieser von der Intensität des einfallenden Lichtes abhängige Spannungsanstieg steuert automatisch die Transmission auf einen tieferen Wert und wirkt damit einer manuellen Hellsteuerung entgegen. Dabei lässt sich die Steuerwirkung in einfacher Weise so bemessen, dass die Lichtintensität, der der Empfänger ausgesetzt wird, ein normiertes, zuträgliches Mass nicht überschreitet. Die Ableitung der Steuerspannung von der Stromversorgungseinheit hat zudem den Vorteil, dass ein zusätzlicher Photosensor, wie er für die durchwegs automatische Regelung der Transmission benötigt wird, entfällt.

Für die Stromversorgung von gesteuerten Lichtschutzfiltern für Schweissarbeiten wird bei bekannten Ausführungen eine Solarzellenanordnung benützt. Bei anderen Ausführungen ist eine galvanische Batterie vorgesehen, welche die Stromversorgung übernehmen kann. Vorzugsweise enthält die Stromversorgungseinheit der erfindungsgemässen Vorrichtung beide Spannungsquellen dieser Art, also eine erste Spannungsquelle mit fester Spannung und eine zweite Spannungsquelle mit variabler, von der Intensität des einfallenden Lichtes abhängiger Spannung. Im Unterschied zu den bekannten Ausführungen können nun aber die beiden Spannungsquellen derart zusammengeschaltet sein, dass die zweite Spannungsquelle die Stromversorgung übernimmt, sobald deren variable Spannung grösser wird als jene der ersten Spannungsquelle. Dies bedeutet, dass bis zu einer bestimmten Intensität des einfallenden Lichts die Steuerspannung für das optoelektronische Filterelement von einer festen Speisespannung abgeleitet wird, während bei höherer Lichtintensität die Steuerspannung um eine von der Lichtintensität abhängige Spannungskomponente erhöht wird. Zu diesem Zweck können z.B. eine galvanische Batterie und ein photoelektrischer Wandler mit gegenseitiger Entkopplung parallelgeschaltet sein.

Auf diese Weise lässt sich die Erfindung mit grösstenteils bereits vorhandenen Mitteln verwirklichen, wobei der bisher als Ersatzbatterie verwendeten galvanischen Batterie eine zusätzliche Funktion zugeordnet wird. Ihre ursprüngliche Funktion bleibt bei dieser Lösung selbstverständlich erhalten. Ausserdem ist es auch in diesem Fall möglich, die galvanische Batterie wie bisher fest in die Vorrichtung einzubauen, d.h. nicht auswechselbar anzuordnen, da der Stromverbrauch auch bei dieser Art der Verwendung äusserst niedrig gehalten werden kann.

In der Zeichnung ist ein Ausführungsbeispiel der Erfindung schematisch dargestellt, und zwar zeigen:

    Fig. 1 ein Blockschema der erfindungsgemässen Steuervorrichtung und

    Fig. 2 das Schaltungsschema der Stromversorgungseinheit.

Die dargestellte Steuervorrichtung, welche für die Steuerung der Transmission eines Lichtschutzfilters für Schweisszwecke vorgesehen ist, unfasst im vorliegenden Beispiel eine Stromversorgungseinheit 1, eine Schalteinheit 2, welche von einem auf Schweisslicht ansprechenden Sensor gesteuert wird, und eine Steuerinheit 3, z.B. in Form eines in der Pulsbreite manuell regelbaren Rechteckspannungsgenerators, zur Steuerung eines optoelektronischen Filterelementes 4, beispielsweise einer Flüssigkristallzelle.

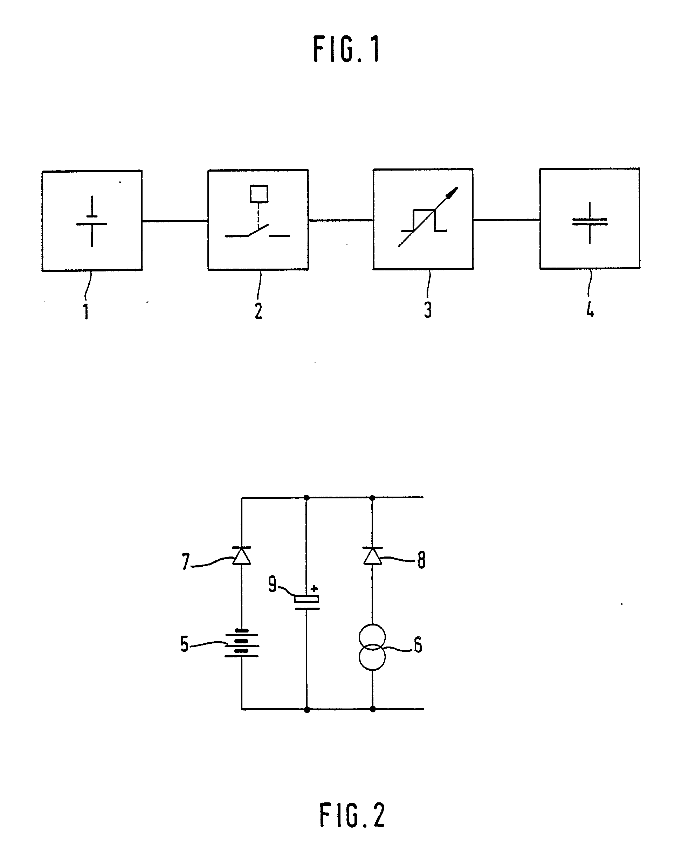

Die Stromversorgungseinheit 1 enthält gemäss Fig. 2 eine galvanische Batterie 5, vorzugsweise eine Lithiumbatterie, und einen photoelektrischen Wandler 6, z.B. eine sogenannte Solarzellenanordnung. Die Batterie 5 und der Wandler 6 sind parallelgeschaltet, wobei in jeden Stromzweig eine Diode 7 bzw. 8 zur gegenseitigen Entkopplung der beiden Spannungsquellen eingeschaltet ist. Zudem ist ein Pufferkondensator 9 vorgesehen.

Solange der photoelektrische Wandler 6, welcher sich z.B. an der Front des Schutzschildes befindet, eine Spannung liefert, deren Wert unterhalb des festen Spannungswertes der galvanischen Batterie 5 liegt, erhält die Steuereinheit 3 ihre Betriebsspannung von dieser Batterie 5, sofern sich die Schalteinheit 2 im Durchlasszustand befindet, also Schweisslicht vorliegt. Dieser Arbeitsbereich erstreckt sich bis zu einem bestimmten Grenzwert der Lichtintensität, bei dem die variable Spannung des Wandlers 6 auf den Wert der festen Spannung der Batterie 5 angewachsen ist. In diesem Arbeitsbereich ist die Transmission des optoelektronischen Filterelements 4 mit Hilfe eines manuell zu betätigenden, die Ausgangsspannung der Steuereinheit 3 beeinflussenden Stellgliedes voll durchsteuerbar.

Bei einer Lichtintensität oberhalb des genannten Grenzwertes übernimmt der photoelektrische Wandler 6 die Stromversorgung zum Betrieb der Steuereinheit und zur Steuerung des optoelektronischen Filterelements 4. Damit setzt die automatische Steuerung des Filterelements 4 ein, welche bewirkt, dass die Transmission desselben mit zunehmender Lichtintensität nicht mehr im selben Mass manuell heraufgesetzt wird, und zwar derart, dass der Aussteuerbereich der manuellen Steuerung in Richtung höherer Transmission zunehmend begrenzt wird.

## Ansprüche

1. Vorrichtung zur Steuerung der Transmission eines Lichtschutzfilters, welches ein optoelektronisches Filterelement aufweist, dadurch gekennzeichnet, dass eine von der Intensität des einfallenden Lichtes unabhängige, manuelle Steuerung und eine von der Intensität des einfallenden Lichtes abhängige, automatische Steuerung des optoelektronischen Filterelementes vorgesehen sind, wobei die automatische Steuerung in einem Bereich oberhalb eines bestimmten Grenzwertes der Lichtintensität wirksam ist, und dass sich die Wirkungen der beiden Steuerungen in diesem Bereich derart überlagern, dass bei Lichtintensitäten über dem Grenzwert die Wirkung der manuellen Steuerung bei einer Betätigung im Sinne einer Erhöhung der Transmission durch die Wirkung der automatischen Steuerung kompensiert wird.

2. Vorrichtung nach Anspruch 1, mit von der angelegten Spannung gegenläufig abhängiger Transmission des optoelektronischen Filterelementes, dadurch gekennzeichnet, dass die Stromversorgungseinheit der Vorrichtung eine die Steuerung des optoelektronischen Filterelementes bewirkende Spannung liefert, deren Wert von einer bestimmten Intensität des einfallenden Lichtes an mit der Lichtintensität zunimmt.

3. Vorrichtung nach Anspruch 2, dadurch gekennzeichnet, dass die Stromversorgungseinheit eine erste Spannungsquelle mit fester Spannung und eine zweite Spannungsquelle mit variabler, von der Intensität des einfallenden Lichtes abhängiger Spannung enthält, und dass die beiden Spannungsquellen derart zusammengeschaltet sind, dass die zweite Spannungsquelle die Stromversorgung übernimmt, sobald deren variable Spannung grösser wird als jene der ersten Spannungsquelle.

4. Vorrichtung nach Anspruch 3, dadurch gekennzeichnet, dass die Stromversorgungseinheit eine galvanische Batterie und einen photoelektrischen Wandler enthält, die mit gegenseitiger Entkopplung parallelgeschaltet sind.

# FIG.1

1    2    3    4

# FIG.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-2 530 039 (CUVELIER et al.) <br> * Seite 1, Zeilen 1-7; Seite 3, Zeile 17 - Seite 4, Zeile 21; Seite 6, Zeile 1 - Seite 11, Zeile 2; Figuren 1-9; Ansprüche 1,3,4 * <br> --- | 1 | G 02 F 1/13 <br> A 61 F 9/06 |
| A | EP-A-0 027 518 (GOR-VUE CORP.) <br> * Figur 7; Seite 15, Zeile 25 - Seite 16, Zeile 16 * <br> ----- | 1 | |

RECHERCHIERTE SACHGEBIETE (Int. Cl.4)

G 02 F
A 61 F

Der vorliegende Recherchenbericht wurde für alle Patentansprüche erstellt

| Recherchenort | Abschlußdatum der Recherche | Prüfer |
|---|---|---|
| DEN HAAG | 11-11-1988 | CONRAD V.HEYDENDORFF K. |